## Europäisches Patentamt

## European Patent Office

## Office européen des brevets

(11) Publication number: **0 509 985 B1**

(12)

# EUROPEAN PATENT SPECIFICATION

(45) Date of publication of patent specification: **13.04.94**

(51) Int. Cl.5: **C12N 1/06**, C12N 9/36, C11D 3/386, //(C12N9/36, C12R1:365)

(21) Application number: **90902767.4**

(22) Date of filing: **11.01.90**

(86) International application number: **PCT/DK90/00009**

(87) International publication number: **WO 91/10723 (25.07.91 91/17)**

(83) Declaration under Rule 28(4) EPC (expert solution)

(54) **BACTERIOLYTIC ENZYME NATIVE TO A NOCARDIOPSIS STRAIN, ITS PRODUCTION AND USE.**

(43) Date of publication of application:
**28.10.92 Bulletin 92/44**

(45) Publication of the grant of the patent:
**13.04.94 Bulletin 94/15**

(84) Designated Contracting States:
**AT BE CH DE DK ES FR GB IT LI LU NL SE**

(56) References cited:
**FR-A- 1 518 511**
**US-A- 3 682 778**
**US-A- 4 828 998**

**Chemical Abstracts, vol. 76, no. 25, 19. June 1972 (Columbus, Ohio, US) s. p. 325 abstract 152061c, & GB, 1268173 (Takeda Chemical Industries, Ltd.)1972**

**Chemical Abstracts, vol. 103, no. 14, 7 October 1985, (Columbus, Ohio, US), Suzuki, Keitarou et al; s. p. 404, abstract 209553x, & Agric. Biol. Chem. 1985, 49(10), 3049-3050**

(73) Proprietor: **NOVO NORDISK A/S**
**Novo Allé**
**DK-2880 Bagsvaerd(DK)**

(72) Inventor: **LIU, Chi-Li**
**41-43 Starr Avenue No. 2**
**Danbury, CT 06811(US)**
Inventor: **BECK, Carol, Marie**
**20 Huntington, Ct**
**Bethel, CT 06801(US)**
Inventor: **OVERHOLT, Janet, M.**
**Unit 17-6**
**166 Old Brookfield Rd.**
**Danbury, CT 06811(US)**

(74) Representative: **Knudsen, Sten Lottrup et al**
**c/o Novo Nordisk A/S,**
**Patent Department,**
**Novo Allé**
**DK-2880 Bagsvaerd (DK)**

Dialog Information Services, File 5, BIOSIS, BIOSIS no. 84018801, accession no. 0017541741, Tsirekidze L G et al: "Actinomycetes of some hot springs in the georgian-ssr USSR and their lytic activity", Soobshch akad nauk gruz ssr 124 (1), 1986 (RECD.1987), 165-168

TIBTECH, vol. 5, 1987, B.A. Andrews et al; s.p. 273-277, s. p. 274 left column line 22-23 and table 2 page 276

Chemical Abstracts, vol. 112, no. 4, 22 January 1990, (Columbus, Ohio, US), s. p. 114, abstract 22800u, & JP, 63234096 (Imai, Satoshi) 1988

Chemical Abstracts, vol. 99, no. 13, 26 September 1983 (Columbus, Ohio, US) s. p. 471, abstract 103739w, & DD,, 200432 (Fleck, Werner et al) 1983

## Description

## TECHNICAL FIELD

The present invention relates to a bacteriolytic enzyme preparation, to a process for preparing a bacteriolytic enzyme preparation, to a microbial culture for use in said process, to a detergent and a deodorant comprising said enzyme preparation and to use of said enzyme preparation.

## BACKGROUND OF INVENTION

The distinct malodorous scent of human adults, popularly called "body odour" has been found to be generated when microorganisms interact with apocrine sweat (J.J. Leyden et al. J.Invest.Dermatology, 1981, 77:413-416). In a number of publications it has been suggested that the common skin microflora is a mixture of micrococcaceae, aerobic diphtheroids and propionic acid bacteria (J.J. Leyden et al. 1981 & J.N. Labows et al J.Soc.Cosmet.Chem. 1982, 34:193-202). The diphtheroids are responsible for the selective generation of the distinctly pungent odors, while the micrococci are responsible for the generation of sweaty, acid odors. The body odour problems in clothes has been of increasing concern because garments made from some synthetics hold odors and because an ever increasing popularity of physical exercise generates many garments permeated with sweat.

The detergent industry has long been using fragrances to make clothes smell fresh and to mask the unpleasant odour of the clothes. Also, "deoperfumes" have been introduced (e.g., into Surf®) to react with odors and prevent them from evaporating and reaching the nose. However, the sources of odour production, i.e., microorganisms in the clothes, are not removed.

In addition to the above mentioned odour generating microorganisms, detergent manufacturers are also concerned about microorganisms found in laundry that might be pathogenic, such as Pseudomonas aeruginosa and Staphylococcus aureus.

Destroying the microorganisms source(s) of body odour, in effect disinfecting the laundry, is believed to be superior approach toward reducing generation of body odour in garments. This result might be achieved during laundering by use of a bacteriolytic enzyme together with the detergent. For any enzyme to be useful in laundry practices as a detergent additive, the enzyme must be active at alkaline pH levels and must not be inhibited by material components in the detergent formulation notably by the surfactant, the builder salts, and any chelating agents present (such as EDTA). Further, such an enzyme must be active towards the relevant microorganisms.

Bacteriolytic enzymes may also be used for destroying harmful microorganisms in food or water, or for processing bacterial cell mass, e.g. for activated sludge treatment, for protoplast formation or for recovery of intracellular products.

Bacteriolytic enzymes are known, including peptidases (such as alanine amidase), glycosidases (such as muramidase or lysozyme) and autolysins (from a number of bacilli and bacteria species), which are capable of depolymerizing peptidoglycan of the microorganism cell wall. Many of the known bacteriolytic enzymes, e.g., Mutanolysin (from Streptomyces globisporus 1829, ATCC 21553) and N-acetylmuramidase (from Streptomyces rutgersensis) have pH optimum between 6-7 and are relatively inactive in the presence of detergent components and/or at alkaline pH levels. Others have little or no activity towards some of the relevant microorganisms.

Bacteriolytic enzymes with high lytic activity at alkaline pH levels (8-10) in the presence of detergent components have not been known heretofore. It is the object of this invention to provide such enzymes.

## SUMMARY OF THE INVENTION

It has now been discovered that certain Nocardiopsis strains elaborate extracellular enzymes capable of hydrolyzing the cell walls of microorganisms present in household laundry, including for example micrococci, Pseudomonas aeruginosa and Staphylococcus aureus. These enzymes are active under laundering conditions, i.e. at alkaline pH levels in the presence of detergent components. Their use during a wash or rinse results in reduced contamination of clothes with common skin microflora, whereby the odour of the dirty clothes can be removed.

Accordingly, the invention provides a bacteriolytic enzyme preparation characterized by comprising a bacteriolytic enzyme native to a Nocardiopsis strain, by having the ability to hydrolyze bacterial cell walls of Micrococcus sedentarius, Pseudomonas aeruginosa and Staphylococcus aureus, by having optimum pH in the range 5-8, by having at least 50 % activity in the pH range 6.3-8.2, and by having optimum temperature in the range 40-60°C. The invention also provides a process for preparing the bacteriolytic enzyme preparation, which comprises cultivating a bacteriolytic enzyme producing strain of Nocardiopsis aerobically under submerged conditions in the presence of carbon and nitrogen sources, and thereafter recovering the enzyme from the culture broth.

A third aspect of the invention provides a biologically pure culture of a bacteriolytic enzyme producing strain of Nocardiopsis.

The invention further provides a detergent composition and a body deodorant comprising said bacteriolytic enzyme preparation. Finally, the invention provides use of said enzyme preparation for hydrolyzing bacterial cell walls.

## BRIEF DESCRIPTION OF DRAWINGS

For further understanding of this invention, reference is made to the attached drawings wherein:

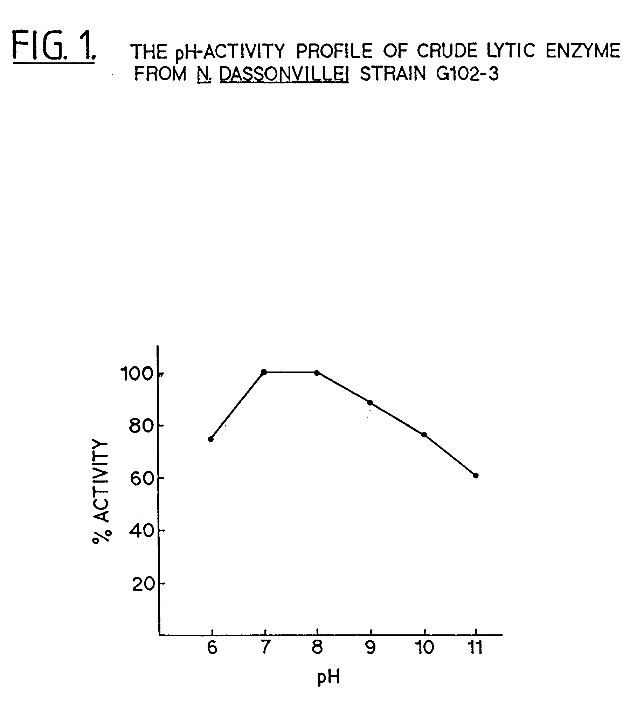

Figure 1 graphically presents the lytic activity of crude enzyme broth from strain D38-3 towards Pseudomonas aeruginosa cells as a function of pH.

## DETAILED DESCRIPTION OF THE INVENTION

### The Microorganisms

Bacteriolytic enzymes of this invention are elaborated extracellularly by atypical Nocardiopsis dassonvillei strains productive of lytic enzyme. Several lytic enzyme complex producing strains of Nocardiopsis dassonvillei have been isolated. On the other hand the Nocardiopsis dassonvillei type strain ATCC 23218 and the Nocardiopsis mutabilis type strain ATCC 31520 do not elaborate lytic enzyme complexes.

The preferred microorganisms of this invention are aerobic, lytic enzyme producing actinomycete isolates of Nocardiopsis dassonvillei.

Three such strains have been deposited for patenting purposes by the inventors at the Agricultural Research Culture Collection (NRRL), Peoria, IL, U.S.A., under the terms of the Budapest Treaty, as follows:

| Depositor's reference | G102-3 | G119-6 | D38-3 |
| --- | --- | --- | --- |
| Deposit No. | NRRL 18349 | NRRL 18350 | NRRL 18364 |
| Deposit date | 24 March, '88 | 24 March, '88 | 20 April, '88 |
| Taxonomic designation | Nocardiopsis dassonvillei | Nocardiopsis dassonvillei | Nocardiopsis dassonvillei |

Temperature for growth of the above described strains is 25°C to 35°C, with poor growth occurring at or above 35°C. Optimal pH for growth of strain G102-3 is 7 and is 8.5-9 for strains G119-6 and D38-3. No growth occurs at or below pH 7.0 for strains G119-6 and D38-3.

On nutrient agar slants, mature colonies of strain G119-6 exhibit mealy aerial mycelia with a faint creamy-yellow tint; as for strain D38-3, the mature colonies show a pinkish-beige cast. On Bennett's agar slants, mature colonies of strain G102-3 have rough, white to cream aerial mycelia.

Lytic enzyme producing mutants and variants of these strains are also within the scope of the invention, as is production of lytic enzyme native to those strains from transformed host cells of other microorganism species (transformed by the recombinant DNA techniques known in the art).

### Production of lytic enzyme

The Nocardiopsis strains of the invention may be cultivated under aerobic conditions in a nutrient medium containing assimilable carbon and nitrogen together with other essential nutrients, the medium being composed in accordance with the principles of the known art. Submerged fermentation is preferred.

Suitable carbon sources are carbohydrates, such as sucrose, glucose, and maltose, or carbohydrate containing materials such as cereal grains, malt, rice and sorghum. The carbohydrate concentration incorporated in the medium may vary widely, e.g. 1 to 15%, but usually 8-10% will be suitable, the percentage being calculated as equivalents of glucose.

The nitrogen source in the nutrient medium could be of an organic or inorganic nature. Among the organic nitrogen sources, quite a number are regularly used in fermentation processes involving the cultivation of actinomycetes. Illustrative examples are soybean meal, cotton seed meal, peanut meal, corn steep liquor, and yeast extract. In addition, the nutrient medium should also contain the usual trace substances.

Since strains G119-6 and D38-3 of the invention are alkalophilic, the cultivation is conducted preferably at alkaline pH (8.5 - 9.0). The alkaline pH may be obtained by addition of suitable buffers, such as sodium carbonate or mixtures of sodium carbonate and sodium bicarbonate (after sterilization of the growth medium). For aerobic submerged cultivation of strains in tank fermentors, it is necessary to use artificial aeration. The rate of aeration may be that employed in conventional tank fermentation.

After fermentation, a liquid enzyme product may be produced from the fermentation broth by removal of coarse material from the broth and, if desired, through concentration of the broth by conventional method, e.g. evaporation at low temperature or by ultrafiltration. Finally, preservatives may be added to the concentrate.

As has been pointed out, the bacteriolytic enzyme preparation of this invention can also be prepared by cultivation of a transformed microorganism cell which is made to contain a gene encoding for and expressing a lytic enzyme native to Nocardiopsis dassonvillei, e.g. to one of the strains herein described, followed by recovery of the lytic enzyme from the culture broth. Thus, the microorganism to be cultivated is either a lytic enzyme producing strain of Nocardiopsis dassonvillei wherein the enzyme is a native enzyme (including mutants and variants of a wild strain productive of the lytic enzyme complex), or is a transformed host organism wherein the gene for the lytic enzyme has been inserted by recombinant DNA techniques. Such techniques are known in the art and generally comprise the following steps:

a) providing a suitable recombinant DNA cloning vector comprising DNA-sequences encoding functions facilitating gene expression and a DNA-sequence encoding a Nocardiopsis dassonvillei lytic enzyme;

b) transforming a suitable host organism with the cloning vector from step a); and

c) culturing the transformed host in a suitable culture medium and recovering the lytic enzyme from the culture medium.

Preferred host organisms are strains of Nocardiopsis, Streptomyces yeast, Aspergillus and Bacillus. It is especially preferred to use A. oryzae as the host according to the teaching in EP 238,023 (Novo).

Properties of lytic enzyme

For the enzyme complex from G102-3, at least 50% of the activity at pH 5.5 (in 0.05M succinate) is seen at pH 8.2 (in 0.05M TRIS) at 30°C with Staphylococcus aureus as substrate.

The temperature optimum of the enzyme complex from G102-3 is about 50°C (in the range 40-60°C) as measured at pH 7 in 0.05M sodium phosphate buffer towards Staph. aureus.

The pH optimum of the enzyme preparation from D38-3 is about 7.5 (in the range of 7-8) in 0.05M phosphate or MOPS buffer with Pseudomonas aeruginosa as substrate at 30°C. At least 80% of the activity at pH 7.5 (in 0.05M phosphate) is seen at pH 9 (in 0.05M TRIS or borate buffer) with Pseudomonas aeruginosa as substrate.

The temperature optimum of the enzyme preparation from D38-3 on Pseudomonas aeruginosa as substrate is 60°C at pH 7 in 0.05M sodium phosphate, with 80% activity seen at 50°C (70°C was not tested because of the ability of this substrate).

The enzyme preparations of the invention have optimum pH in the range 5-8 (at 30°C) and have at least 50% lytic activity in the pH range 6.3-8.2. They have temperature optimum in the range 40-60°C (measured at pH 7 in 0.05M phosphate buffer).

As shown in Table I, hereinafter provided the lytic enzyme complex from Nocardiopsis dassonvillei strain G102-3 (NRRL 18349) shows excellent activity towards the target organisms both in pH 7.0 buffer and in pH 9.5 buffer. Advantageously, the lytic enzyme complex from strain G102-3 exhibits activity towards the target organisms at lower temperature of 15°C as well as at 40°C, which makes this lytic enzyme complex advantageous for low temperature laundering application, or room temperature rinse water application.

Table II shows that the lytic enzyme complex from strain G102-3 exhibits good lytic activity towards substrates in the presence of detergent components. Meanwhile, lytic enzyme from strain D38-3 shows excellent activity towards Pseudomonas aeruginosa in the presence of detergent components. More specifically, the enzyme preparations of the invention have bacteriolytic activity in 1.5 g/l detergent solution at least equal to the activity in buffer at the same pH.

The data in Table I compared to the data in Table III and that in Table II with Table IV, demonstrates that the decrease in cell suspension turbidity correlates roughly with the true bacterial survival counts both in buffers and in detergent solutions.

The data presented in Table VI indicates that in the presence of detergent components Alcalase® alone had some lytic effect, and the addition of the lytic enzyme complex of this invention increased the lysis to 75-93%.

As illustrated by the data in Table VIII, the enzyme preparations of the invention are active towards a wide range of bacteria, including a number of bacteria whose removal is desirable for personal care or food hygiene, e.g. Micrococci, Corynebacteria, E. coli, Vibrio and Salmonella. Such bacteria as Micrococcus kristinae and Streptococcus faecium are lysed by the enzyme complex from G102-3.

Enzyme Preparation

Solid enzyme preparations may be prepared from the purified and/or concentrated broth by precipitation with salts such as $Na_2SO_4$ or with water miscible solvents such as ethanol or acetone. Removal of the water in the fermentation broth by suitable drying methods such as spray drying, evaporation under vacuum or even lyophilization may also be employed. The hydrolytic activity of lytic enzyme preparations so obtained is usually in the range of 200 to 5000 units/g of powder. This crude product may be (partially) purified if enzyme concentrates of greater unit activity are desired in the market place. A suitable activity range for a detergent additive containing the lytic enzyme of this invention is 50,000 to 1 million units per gram of additive (solid form or liquid form).

Typical detergent additive forms known in the art may be employed, particularly a non-dusting granulate, a stabilized liquid or a protected enzyme.

Non-dusting granulates may be produced, e.g according to U.S. 4,106,991 or U.S. 4,661,452 and the granules may be coated according to principles known in the art.

Liquid form lytic enzyme preparations may be stabilized, e.g. by addition of propylene glycol, other polyols, sugars, sugar alcohols and boric acid or by other enzyme stabilizers known in the art.

A particularly advantageous feature of the present invention is that the lytic enzyme is compatible with and is most useful in combination with alkaline Bacillus proteinases and in particularly with the commercially available alkaline Bacillus proteases commercially offered to and used by the soapers, e.g., Alcalase®, Esperase®, Savinase®, Maxatase®. Together protease and the lytic enzyme generate a combined, and perhaps synergistic bacteriolytic effect. Laundering tests on some target microorganisms using combinations of an alkaline Bacillus protease and a lytic enzyme complex has resulted in more than a 90% kill level. A detergent additive which comprises a protease/lytic enzyme mixture is a preferred product mode of the invention.

Component enzymes

N. dassonvillei strain G102-3 produces a complex of at least two lytic enzymes. The enzyme complex produced by G102-3 may be separated into the two main component lytic enzymes, designated enzyme A and B, by CM-Sephadex ion exchange chromatography. Enzyme A has a molecular weight of 24,000 and an isoelectric point of 8.3, whereas enzyme B has MW 26,000 and pI greater than or equal to 9.5. Both of these enzymes generate reducing ends from Staphylococcus aureus peptidoglycan, which is indicative of N-acetylhexosaminidase activity.

Detergent composition

The detergent compositions employed in practice of the invention are comprised of surfactants known in the art which may be of the anionic, non-ionic, cationic or zwitterionic type, or a mixture of these. Typical examples of anionic surfactants are linear alkyl benzene sulfonate (LAS), alpha olefinsulfonate (AOS), alcohol ethoxy sulfate (AES) and natural soap of alkali metals.

Detergents compositions employed in practice of the invention, may contain other detergent ingredients known in the art, such as builders, bleaching agents, bleach activators, anti-corrosion agents, sequestering agents, anti-soil redeposition agents, perfumes, stabilizers for the enzymes and so on.

The detergent compositions may be formulated in any convenient form, such as powders, liquids, etc. The lytic enzyme may be stabilized in a liquid detergent by inclusion of enzyme stabilizers in the formulation, e.g. those mentioned above.

Most detergent compositions exhibit a pH in solution of 8-10.5. Due to its broad pH optimum, lytic enzyme of the invention is highly active in this entire range, as shown in Figure 1 and 2.

The detergent formulation employed in practice of this invention may include one or more other detergent enzymes in addition to lytic enzyme of the invention. Examples are protease, lipase, amylase and cellulase. Presence of protease is, of course, preferred.

The lytic enzymes of this invention (and the alkaline Bacillus proteases as well) are compatible with most commercially available detergent compositions formulations, with the proviso that their employment in detergent formulations containing some bleaches and those which create a wash water pH exceeding pH 11 might not be practical. The amount of enzyme additive is generally from 0.5-5% by weight of the detergent formulation.

Thus, the detergent additive form of the lytic enzymes of this invention fits into a well defined niche in the art, namely, as a concentrate of about 50,000 to 1 million units per gram for incorporation into a (soaper's) detergent formulation as 0.5-5% by weight of volume thereof so as to generate a lytic enzyme concentration of about 1000 to 20,000 units, preferably 2000 to 10,000 units per liter in the wash water. Comparably for direct addition into detergent containing wash water or into a rinse water free of detergent the additive may be supplied to consumers to generate the ultimate desired concentration, e.g. 2000 to 10,000 units per liter.

In a preferred mode of the invention an alkaline Bacillus protease in concentration of 0.5 to 3.0 Anson units per gram of additive, (or if more conveniently measured thereby an activity 0.5-3.0 KNPU/g) may be included in the lytic enzyme mixture additive supplied to the soapers for inclusion in their detergent formulations, or alternatively to consumers for a separate addition to wash or rinse water. A protease containing additive may, of course, be added to the wash or rinse water separately from the lytic enzyme additive. In any event, concentrations of 2000-10,000 units per liter of lytic enzyme and of 0.01-0.15 Anson units per liter of protease in wash or rinse water are preferred, and most preferably 0.02-0.15 AU/l. The enzyme mixture results in a combined or a synergistic improvement in the kill ratio of body odour generating microflora.

## Hydrolysis of bacterial cell walls

The enzyme preparation of the invention is useful for reducing the number of undesired bacteria. Thus, it may be used in food applications (as a food preservative or for disinfection during food processing) to control such organisms as Listeria, E. coli, Salmonella, Vibrio and Campylobacter. It may also be used in water treatment, e.g. in hospitals and in industrial cooling water towers, to control Legionella. Another such use is disinfection of hospital instruments, particularly those which cannot withstand sterilizing temperature, where control of Staphylococcus aureus, Pseudomonas aeruginosa and Campylobacter is important.

Further, the enzyme preparation of the invention may be used for lysing bacterial cell mass in a laboratory or industrial setting. Thus, it may be used to treat activated sludge or to work as a dewatering aid for sludge. It may also be used as a research enzyme for protoplast formation. And it may be used as a cell-opening aid to recover products produced intracellularly in bacteria, e.g. cloned products such as enzymes.

## EXAMPLES

For further understanding of the invention the following specific examples are provided.

## Assay for Cell Wall Hydrolytic Activity

In the examples, the cell wall hydrolytic activity in strain G102-3 and strain G119-6 and strain D38-3 cultures was determined by the turbidity reduction method (K. Hayashi et. al. Agric. Biol. Chem. 1981. 45-(10):2289-2300). Viable or lyophilized target organisms, Micrococcus kristinae (ATCC 27570), Micrococcus sedentarius (ATCC 14392), Pseudomonas aeruginosa (ATCC 9027) and Staphylococcus aureus (ATCC 6538), are first suspended in 62.5mM phosphate buffer, pH 7.0 to an OD at 660 nm of 0.8. To 2 ml of such a cell suspension, 0.5 ml of an appropriately diluted enzyme broth is added and the reaction mixture is incubated at 15°C or 40°C for 10 minutes. At the end of incubation time, the decrease in turbidity of cell suspension at 660 nm (Δ OD 660 nm) is measured by use of a spectrophotometer. One unit is defined as the amount of lytic enzyme which causes a decrease of 0.001 at OD 660 nm in turbidity of the cell suspension at said temperature per minute.

It should be appreciated that measurement of different lytic enzyme preparations against different test microorganisms can be expected to provide widely varying values for cell wall hydrolytic activity, and a high degree of variability has been found to exist. To avoid confusion the numerical values hereinafter provided for the cell wall hydrolytic activity will be those measured by the herein described test in tests against Staphylococcus aureus, (except of course when a different target microorganism is named). The inventors hereof recognize that the unit values they report are somewhat artificial and note that any lytic enzyme preparation native to a strain of Nocardiopsis dassonvillei not exemplified herein should be tested against many target microorganisms to ascertain effectiveness.

Cell count experiments have ascertained to the satisfaction of the inventors hereof that the turbidity decrease in cell suspension at 660 nm correlates well with the actual kill of the target organism. The procedure is the same as described by K. Hayashi et al., supra, except that all solutions excluding cell suspension are autoclaved and lytic enzyme solution is filter sterilized. At the end of incubation, reaction mixtures are serially diluted and plated on nutrient agar plates for survival bacterial counts.

Cell wall hydrolytic activity was also determined by the chemical, enzymatic assays.

(a). N-acetylmuramidase activity is measured by using cell wall of Staphylococcus aureus as the substrate and following the formation of N-acetylhexosamine which is released from the cell wall. To 1 ml of Staphylococcus aureus cell wall suspension (which contains 1.6 mg cell wall) made in 50mM MES buffer, pH 6.0, 0.2 ml enzyme solution is added and the reaction mixture is incubated at 37°C for 30 minutes with shaking. At the end of incubation time, the unused cell wall is removed by centrifugation and the supernatant is used to measure the concentration of released N-acetylhexosamine via p-dimethylaminobenzaldehyde (DMAB) method (J.L. Reissig et al. Biol. Chem. 1955, 217:959-966). One unit is the amount of enzyme which releases 1 nmole N-acetylhexosamine from the cell wall at 37°C per minute.

(b). Chitinase activity is measured by using chitin as the substrate and following the formation of N-acetylglucosamine in solution. 0.5 ml enzyme solution is mixed with a 0.5 ml chitin suspension which is composed of 4 mg chitin/ml in 0.1 M citric acid/0.2M $Na_2HP0_4$ buffer, pH 6.5. The reaction mixture is then incubated at 37°C for 90 minutes with vigorous shaking. At the end of incubation, the unused chitin is removed by centrifugation and the supernatant is then analyzed for N-acetylglucosamine concentration by DMAB method.

(c). Laminarinase activity is assayed by using laminarin as the substrate and following the increase of the reducing sugar concentration. Reaction mixtures are comprised of 0.1 ml laminarin (15 mg/ml in 0.1 M citric acid/0.2M $Na_2HP0_4$ buffer, pH 6.0), 0.4 ml buffer and 0.2 ml enzyme solution. Mixtures are incubated for 10 minutes at 37°C. Then the reaction is terminated by addition of 0.3 ml cold $H_20$ and cooled to room temperature in cold water. An aliquot (200 ul) of the solution is then used to measure the concentration of the reducing sugar via the micro Nelson method (R.G. Spiro. Method. Enzymology, 1966, Vol. 8:p.3).

**EXAMPLE I**

Nocardiopsis dassonvillei strain G102-3 (NRRL 18349) was cultivated at 30°C on a rotary shaking table (250 rpm) in 250 ml triple-baffled Erlenmeyer flasks containing 50 ml of medium of the following composition:

Composition of the medium in grams per liter:

| Maltodextrin M-100 | 20 |
| Soy bean meal | 20 |
| Yeast extract | 5 |
| NaCl | 2 |

Before sterilization, the pH of the medium was adjusted to 7.0 by the addition of a few drops of 0.1 M Na0H. After 2 to 4 days of incubation, the lytic enzyme activity of the broth was determined by using the turbidity reduction method described above. The lytic activity of the G102-3 broth was 16.2 unit/ml with Staphylococcus aureus as the substrate after 72 hours incubation.

Nocardiopsis dassonvillei strain G119-6 (NRRL 18350) and strain D38-3 (NRRL 18364) were also cultivated at 30°C as described, except for the following differences:

Composition of the medium in grams per liter:

| Maltodextrin M-100 | 20 |
| Soy bean flour | 20 |
| Yeast extract | 2 |
| $K_2HP0_4$ | 1 |
| $MgS0_4 \cdot 7H_20$ | 1 |

After sterilization, the pH of the medium was adjusted to 8.5-9.0 by the addition of 5 ml of 1M solution of sodium carbonate/sodium bicarbonate buffer, pH 9.2. After 114 hours of incubation, the broth of strain G119-6 had a lytic activity of 17.8 unit/ml with the viable Staphylococcus aureus as the substrate. After 142 hours of incubation, the broth of strain D38-3 had a lytic activity of 47.5 unit/ml with the viable Pseudomonas aeruginosa as the substrate.

**EXAMPLE II**

The lytic activity of strain G-102-3 lytic enzyme from Example I is depicted in Table 1 when different microorganisms were used as the substrates. The target organisms, Micrococcus kristinae, Micrococcus sedentarius, Pseudomonas aeruginosa and Staphylococcus aureus were suspended in 62.5mM phosphate buffer, pH 7.0 and 50mM borate buffer, pH 9.5 to give an initial OD at 660 nm of 0.8. Lytic reactions were carried out with 3 units per ml of the reaction mixture at 15°C and 40°C with 10 minutes incubation. At the end of incubation, the reduction of turbidity of the cell suspensions was measured at 660 nm by use of a spectrophotometer.

Table I

| Substrate organism | | Δ OD 660 nm | |
|---|---|---|---|
| | | at 15°C | at 40°C |
| M. kristinae | in pH 7 buffer<br>in pH 9.5 buffer | 0.181<br>0.154 | 0.174<br>0.155 |
| M. sedentarius | in pH 7 buffer<br>in pH 9.5 buffer | 0.237<br>0.235 | 0.148<br>0.123 |
| Pseud. aeruginosa | in pH 7 buffer<br>in pH 9.5 buffer | 0.264<br>0.224 | 0.160<br>0.161 |
| Staph. aureus | in pH 7 buffer<br>in pH 9.5 buffer | 0.289<br>0.272 | 0.169<br>0.147 |

**EXAMPLE III**

The lytic activity of strain G102-3 lytic enzyme and D38-3 lytic enzyme (from Example I) in the presence of detergent is depicted in Table II when different microorganisms were used as the substrates. The target organisms, Micrococcus kristinae, Micrococcus sedentarius, Pseudomonas aeruginosa, and Staphylococcus aureus were suspended in detergent solution which was made by addition of 1.5 g detergent powder into 1 l of deionized $H_2O$ and then adjusted to 9° dH German hardness by addition of $CaCl_2$ and $MgCl_2$. The detergent formulation used in the tests was Tide® with no phosphate.

Lytic reactions were carried out at 15°C and 40°C with 10 minutes incubation at 3 units per ml of the lytic enzyme complex. At the end of incubation, the reduction of turbidity of the cell suspensions was measured at 660 nm by use of a spectrophotometer.

Table II

| Substrate organism | Δ OD 660 nm | | | |
|---|---|---|---|---|
| | G102-3 enzyme | | D38-3 enzyme | |
| | at 15°C | at 40°C | at 15°C | at 40°C |
| M. kristinae | 0.218 | 0.188 | 0 | 0 |
| M. sedentarius | 0.242 | 0.159 | 0.031 | 0.087 |
| Pseud. aeruginosa | 0.233 | 0.166 | 0.324 | 0.495 |
| Staph. aureus | 0.372 | 0.252 | 0.069 | 0.231 |

**EXAMPLE IV**

To assess the actual number of microorganisms which were lysed by lytic enzyme produced from strain G102-3, the following viable cell count experiments were carried out and the results are shown in Table III. Overnight-grown substrate organisms, Micrococcus kristinae and Staphylococcus aureus, were suspended in 50mM borate buffer, pH 9.5 to - $10^4$ CFU/ml. To 2 ml of cell suspension, 0.5 ml of appropriately diluted enzyme solution (to 3 units/ml of reaction mixture) was added and incubated at 15°C or 40°C for 10 minutes with periodic mixing. All the solutions including enzyme were sterile. At the end of incubation, the reaction mixtures were serially diluted and plated on nutrient agar plates for survival bacterial counts.

Table III

| Substrate organism | % Kill | |
|---|---|---|
| | at 15°C | at 40°C |
| M. kristinae | 35 | 44 |
| Staph. aureus | 47 | 53 |

**EXAMPLE V**

The actual number of microorganisms which were lysed by lytic enzyme from strain G102-3 at 3 units/ml of reaction mixture in the presence of detergent components (1.5 g/l) was determined by an experiment similar to that in Example IV except that Micrococcus kristinae and Staphylococcus aureus were suspended in the detergent solution to approximately $10^4$ CFU/ml which was described in Example III. The results are shown in Table IV.

Table IV

| Substrate organism | % Kill | |
|---|---|---|
| | at 15°C | at 40°C |
| M. kristinae | 64 | 58 |
| Staph. aureus | 60 | 52 |

It is evident that in buffer or in detergent solution lytic enzyme from strain G102-3 consistently lyses 35-64% of viable microorganisms. The lytic enzyme is most effective in detergent solution.

**EXAMPLE VI**

A comparative lytic activity of lytic enzymes from strain G102-3, Mutanolysin and N-acetylmuramidase from Streptomyces rutgersensis (ATCC 3350) towards target microorganisms in the presence of detergent components is depicted in Table V. Lactobacillus plantarum (ATCC 8014) was the substrate organism for strain G102-3 lytic enzyme and Mutanolysin whereas Streptococcus faecium (ATCC 8043) was the substrate for G102-3 lytic enzyme and Streptomyces rutgersensis (ATCC 3350) enzyme. It is known that Lactobacillus plantarum and Streptococcus faecium are the best target organism for Mutanolysin and N-acetylmuramidase from Streptomyces rutgersensis, respectively. The detergent solution was as described in Example III, and the same 3 units/ml of enzyme activity level was used throughout the experiment while reactions were carried at 15°C.

10

Table V

| Enzyme | Δ OD 660 nm | |
|---|---|---|
| | Lactobacillus plantarum | Streptococcus faecium |
| From strain G102-3 | 0.219 | 0.121 |
| Mutanolysin from S. globisporus | 0.083 | N.D. |
| From S. rutgersensis | N.D. | 0.0 |

**EXAMPLE VII**

The combination effect of Alcalase® and lytic enzyme from strain G102-3 on viable microorganisms was demonstrated in the following experiments.

When Micrococcus kristinae and Staphylococcus aureus were suspended in detergent solution as described in Example V, 0.05 AU/l of Alcalase® was dosed in to examine any additional lytic effect of Alcalase® in detergent solution. As shown in Table VI, Alcalase® alone in detergent does have some lytic effect. However, when lytic enzyme produced by G102-3 was added at 3 units/ml (3000 units/l) in combination with 0.05 AU/l of Alcalase®, an average of 75-93% lysis was achieved.

Table VI

| | M. kristinae | Staph. aureus |
|---|---|---|
| Detergent alone | 0% Kill | 0% Kill |
| Alcalase® + Detergent | 46-51 | 36-65 |
| Lytic enzyme + Alcalase® + Detergent | 75-93 | 85-89 |

An increased dose of Alcalase® (up to 0.2 AU/l) in detergent did not result in a significant increase in lysis of M. Kristinae or S. aureus.

**EXAMPLE VIII**

The synergistic effect of Savinase® or Esperase® with lytic enzyme from strain G102-3 on lysis of Staphylococcus aureus in liquid detergent was demonstrated in the following experiments.

Liquid detergent, Wisk® (alkaline pH solution), was made to the commercial level. The target organism Staphylococcus aureus was suspended directly in the detergent solution to an initial $OD_{660}$ - 0.8. Savinase® or Esperase® was dosed in at the commercial level (0.06 KNPU/l) as described in Example VII. The lytic reaction with 3 units/ml was monitored by the decrease in turbidity at 660 nm. As shown in Table VII, Savinase® or Esperase® alone in liquid detergent has no lytic effect on the organism. It is also evident that G102-3 lytic enzyme expresses good lytic activity in both powder detergent (Example VII) and liquid detergent. A synergistic effect of lytic enzyme from G102-3 with Savinase® or Esperase® on lysis of Staphylococcus aureus seems to have been obtained in the Wisk®.

Table VII

| Conditions | % Lysis | |
|---|---|---|
| | 15°C | 40°C |
| Detergent alone | 0 | 0 |
| Savinase® + Detergent | 0 | 0 |
| Esperase® + Detergent | 0 | 0 |
| Lytic enzyme + Detergent | 0 | 36 |
| Lytic enzyme + Savinase® + Detergent | 7 | 90 |
| Lytic enzyme + Esperase® + Detergent | 3 | 91 |

**EXAMPLE IX**

Microorganisms which are known to be pathogens, opportunists, common skin and/or clothing contaminants and/or difficult to be lysed by egg-white lysozyme were tested as the substrate organisms for strain G102-3 lytic enzyme and strain D38-3 lytic enzyme. Common skin and/or clothing contaminants were isolated in our laboratory and designated as NOVO 1, 8, 12, 13. A comparison was made between the effect of 1 mg/ml of egg-white lysozyme (from Sigma) and that of 1 mg lyophil from crude fermentation broth/ml of reaction mixture. As shown in Table VIII, in most cases lytic enzyme produced by strain G102-3 is definitely much more effective than the egg-white lysozyme, whereas D38-3 lytic enzyme is demonstrated to be extremely potent to Pseudomonas aeruginosa cells.

12

## Table VIII

| Substrate organism | G102-3 lytic enzyme (% lysis) | D38-3 lytic Enzyme (% lysis) | egg-white lysozyme (% lysis) |
|---|---|---|---|
| Lactobacillus plantarum (ATCC 8014) | 50 | 8 | 0 |
| Micrococcus kristinae (ATCC 27570) | 20 | 0 | 2 |
| Micrococcus sedentarius (ATCC 14392) | 30 | 11 | 0 |
| Pseudomonas aeruginosa (ATCC 9027) | 98 | 99 | 0 |
| Streptococcus faecium (ATCC 8043) | 29 | 0 | 2 |
| Staphylococcus aureus (ATCC 6538) | 35 | 19 | 0 |
| Staphylococcus aureus (NOVO-1) | 47 | 2 | 7 |
| Micrococcus epidermidis (NOVO-8) | 4 | 7 | 0 |
| Micrococcus sp. (NOVO-12) | 4 | 12 | 0 |
| Micrococcus sp. (NOVO-13) | 13 | 0 | 0 |
| Campylobacter fetus (ATCC 27374) | 26 | ND | 0 |
| Corynebacterium liquefaciens (ATCC 14929) | 28 | 22 | 20 |
| Eschericia coli (ATCC 26) | 52 | 27 | 0 |
| Klebsiella pneunomiae (ATCC 13882) | 9 | ND | 0 |
| Legionella pneumophila (ATCC 33152) | 8 | ND | 0 |

| Listeria innocua | 6 | ND | 0 |
| Salmonella arizona (ATCC 12323) | 37 | 17 | 0 |
| Streptococcus lactis (ATCC 11454) | 34 | ND | ND |
| Vibrio parahaemolyticus (ATCC 35117) | 29 | 29 | 0 |

**EXAMPLE X**

The lytic enzyme produced by strain G102-3 was identified as a mixture of enzymes, namely N-acetylmuramidase, chitinase and laminarinase, whereas the lytic enzyme produced by strain D38-3 contained chitinase and laminarinase.

Their individual enzyme activity from fermentation broth of Example I are tabulated in Table IX.

Table IX

|  | G102-3 enzyme (u/l) | D38-3 enzyme (u/l) |
| --- | --- | --- |
| N-acetylmuramidase | 12 | ND |
| Chitinase | 5.0 | 0.33 |
| Laminarinase | 80 | 70 |

**Claims**

1. A bacteriolytic enzyme preparation characterized by comprising a bacteriolytic enzyme derivable from a Nocardiopsis strain, by having the ability to hydrolyze bacterial cell walls of Micrococcus sedentarius, Pseudomonas aeruginosa and Staphylococcus aureus, by having optimum pH in the range 5-8, by having at least 50 % activity in the pH range 6.3-8.2, and by having optimum temperature in the range 40-60°C.

2. The enzyme preparation of Claim 1, wherein the Nocardiopsis strain is a strain of N. dassonvillei, preferably NRRL 18349, NRRL 18350 or NRRL 18364.

3. The enzyme preparation of Claim 1 or 2, having optimum pH in the range 7-8 and having an activity at pH 9 which is at least 80 % of the activity at pH 7.5.

4. The enzyme preparation of any preceding Claim, further characterized by the ability to hydrolyze bacterial cell walls of Micrococcus kristinae.

5. The enzyme preparation of Claim 4, wherein the Nocardiopsis strain is N. dassonvillei NRRL 18349.

6. The enzyme preparation of Claim 5, comprising a lytic enzyme having a molecular weight of 24,000 or 26,000 and an isoelectric point of 8.3 or at least 9.5, respectively.

7. The enzyme preparation of any preceding Claim, which further comprises an alkaline Bacillus protease.

8. The mixed enzyme preparation of Claim 7 comprising from 50,000 to 1 million units of bacteriolytic enzyme per gram of additive and from 0.5 to about 3.0 Anson units of protease per gram of additive.

9. The enzyme preparation of any preceding Claim in the form of a detergent additive, preferably in the form of a non-dusting granulate or a stabilized liquid.

10. A process for producing the bacteriolytic enzyme of any preceding claim, which comprises cultivating a bacteriolytic enzyme producing strain of Nocardiopsis under aerobic conditions in a nutrient medium containing assimilable sources of carbon, nitrogen, and phosphorus, and thereafter recovering the enzyme from the culture broth.

11. A process according to Claim 10, whereby the strain belongs to N. dassonvillei.

12. A process according to Claim 11, whereby the strain is NRRL 18349, NRRL 18350, NRRL 18364 or a mutant or variant thereof.

13. A biologically pure culture of a Nocardiopsis strain capable of producing the bacteriolytic enzyme of claim 1, 3, 4 or 6.

14. The culture of Claim 13, whereby the strain belongs to N. dassonvillei.

15. A culture according to Claim 14 of strain NRRL 18349, NRRL 18350, NRRL 18364 or a mutant or variant thereof.

16. A detergent composition comprising the bacteriolytic enzyme preparation of any of Claims 1 - 9.

17. A detergent composition according to Claim 16, comprising 1000 - 20,000 units of bacteriolytic enzyme per gram of detergent.

18. A detergent composition according to Claim 16 or 17, further comprising 0.001 to 0.5 Anson units of alkaline Bacillus protease per gram of detergent.

19. Use of the enzyme preparation of any of Claims 1 - 9 for hydrolyzing bacterial cell walls.

20. Use according to Claim 19 for reducing the count of harmful bacteria.

21. Use according to Claim 20 as a food preservative, for disinfection in food processing, in water treatment, in disinfection of hospital instruments or for reducing the body odour of clothes.

22. Use according to Claim 20 for reducing body odour of clothes by washing or rinsing the clothes in a detergent containing wash water or in a detergent free rinse water containing the enzyme preparation.

23. Use according to any of Claims 19-22, whereby the wash or rinse water contains 1000 - 20,000 units of bacteriolytic enzyme per liter.

24. Use according to any of Claims 19-23, whereby the wash or rinse water further comprises an alkaline Bacillus protease, preferably in an activity level of 0.02 - 0.15 Anson units per liter.

25. Use according to Claim 19 in processing of bacterial cell mass.

26. Use according to Claim 25 in treatment of activated sludge, in protoplast formation or in recovery of intracellularly secreted compounds.

27. A body deodorant comprising the bacteriolytic enzyme preparation of any of Claims 1 - 9.

**Patentansprüche**

1. Eine bakteriolytische Enzymzubereitung, dadurch gekennzeichnet, daß sie ein aus einem Nocardiopsis-Stamm gewinnbares bakteriolytiches Enzym umfaßt, daß sie die Fähigkeit hat, bakterielle Zellwände von Micrococcus sedentarius, Pseudomonas aeruginosa und Staphylococcus aureus zu hydrolysieren, daß sie einen optimalen pH im Bereich 5-8 hat, daß sie wenigstens 50% Aktivität im pH-Bereich 6,3-8,2 hat und daß sie eine optimale Temperatur im Bereich 40-60°C hat.

2. Die Enzymzubereitung nach Anspruch 1, wobei der Nocardiopsis-Stamm ein Stamm von N. dassonvillei ist, vorzugsweise NRRL 18349, NRRL 18350 oder NRRL 18364.

3. Die Enzymzubereitung nach Anspruch 1 oder 2, die einen optimalen pH im Bereich 7-8 hat und eine Aktivität bei pH 9 hat, die Wenigstens 80% der Aktivität bei pH 7,5 ist.

4. Die Enzymzubereitung nach irgendeinem vorangehenden Anspruch, außerdem gekennzeichnet durch die Fähigkeit, bakterielle Zellwände von Micrococcus kristinae zu hydrolysieren.

5. Die Enzymzubereitung nach Anspruch 4, wobei der Nocardiopsis-Stamm N. dassonvillei NRRL 18349 ist.

6. Die Enzymzubereitung nach Anspruch 5, die ein lytisches Enzym mit einer relativen Molekülmasse von 24.000 bzw. 26.000 und einem isoelektrischen Punkt von 8,3 bzw. wenigstens 9,5 umfaßt.

7. Die Enzymzubereitung nach irgendeinem vorangehenden Anspruch, die außerdem eine alkalische Bacillus-Protease umfaßt.

8. Die Mischenzymzubereitung nach Anspruch 7, die von 50.000 bis 1 Millionen Einheiten bakteriolytisches Enzym pro Gramm Zusatzstoff und von 0,5 bis etwa 3,0 Anson-Einheiten Protease pro Gramm Zusatzstoff umfaßt.

9. Die Enzymzubereitung nach irgendeinem vorangehenden Anspruch in der Form eines Waschmittelzusatzstoffes, vorzugsweise in der Form eines nicht-staubenden Granulates oder einer stabilisierten Flüssigkeit.

10. Ein Verfahren zur Herstellung des bakteriolytischen Enzyms nach irgendeinem vorangehenden Anspruch, welches Kultivieren eines ein bakteriolytisches Enzym produzierenden Stammes von Nocardiopsis unter aeroben Bedingungen in einem Nährstoffmedium, das assimilierbare Kohlenstoff-, Stickstoff- und Phosphorquellen enthält, und anschließendes Gewinnen des Enzyms aus der Kulturbrühe umfaßt.

11. Ein Verfahren nach Anspruch 10, wobei der Stamm zu N. dassonvillei gehört.

12. Ein Verfahren nach Anspruch 11, wobei der Stamm NRRL 18349, NRRL 18350, NRRL 18364 oder eine Mutante oder Variante derselben ist.

13. Eine biologisch reine Kultur eines Nocardiopsis-Stammes, der in der Lage ist, das bakteriolytische Enzym nach Anspruch 1, 3, 4 oder 6 zu produzieren.

14. Die Kultur nach Anspruch 13, wobei der Stamm zu N. dassonvillei gehört.

15. Eine Kultur nach Anspruch 14 von Stamm NRRL 18349, NRRL 18350, NRRL 18364 oder einer Mutanten oder Varianten derselben.

16. Eine Waschmittelzusammensetzung, die die bakteriolytische Enzymzubereitung nach einem der Ansprüche 1-9 umfaßt.

17. Eine Waschmittelzusammensetzung nach Anspruch 16, die 1000-20.000 Einheiten bakteriolytisches Enzym pro Gramm Waschmittel umfaßt.

18. Eine Waschmittelzusammensetzung nach Anspruch 16 oder 17, die außerdem 0,001 bis 0,5 Anson-Einheiten alkalische Bacillus-Protease pro Gramm Waschmittel umfaßt.

19. Verwendung der Enzymzubereitung nach einem der Ansprüche 1-9 zum Hydrolysieren bakterieller Zellwände.

EP 0 509 985 B1

**20.** Verwendung nach Anspruch 19 zur Verringerung der Zahl schädlicher Bakterien.

**21.** Verwendung nach Anspruch 20 als ein Nahrungsmittelkonservierungsstoff, zur Desinfektion bei der Nahrungsmittelverarbeitung, bei der Wasserbehandlung, bei der Desinfektion von Krankenhausinstrumenten oder zur Verringerung des Körpergeruchs von Kleidung.

**22.** Verwendung nach Anspruch 20 zur Verringerung des Körpergeruchs von Kleidung durch Waschen oder Spülen der Kleidung in einem Waschmittel enthaltenden Waschwasser oder in einem waschmittelfreien Spülwasser, das die Enzymzubereitung enthält.

**23.** Verwendung nach einem der Ansprüche 19-22, wobei das Wasch- oder Spülwasser 1000-20.000 Einheiten bakteriolytisches Enzym pro Liter enthält.

**24.** Verwendung nach einem der Ansprüche 19-23, wobei das Wasch- oder Spülwasser außerdem eine alkalische <u>Bacillus</u>-Protease umfaßt, vorzugsweise in einem Aktivitätsniveau von 0,02-0,15 Anson-Einheiten pro Liter.

**25.** Verwendung nach Anspruch 19 bei der Verarbeitung von bakterieller Zellmasse.

**26.** Verwendung nach Anspruch 25 bei der Behandlung von Aktivschlamm, bei der Protoplastenbildung oder bei der Gewinnung von intrazellulär sekretierten Verbindungen.

**27.** Ein Körperdeodorant, das die bakteriolytische Enzymzubereitung nach einem der Ansprüche 1-9 umfaßt.

**Revendications**

**1.** Préparation enzymatique bactériolytique, caractérisée par le fait qu'elle comprend une enzyme bactériolytique pouvant dériver d'une souche de *Nocardiopsis*, qu'elle est capable d'hydrolyser les parois cellulaires bactériennes de *Micrococcus sedentarius*, *Pseudomonas aeruginosa* et *Staphylococcus aureus*, qu'elle a un optimum de pH compris entre 5 et 8, qu'elle a au moins 50 % d'activité dans l'intervalle de pH 6,3-8,2 et qu'elle a une température optimale comprise entre 40 et 60° C.

**2.** Préparation enzymatique selon la revendication 1, dans laquelle la souche de *Nocardiopsis* est une souche de *N. dassonvillei,* de préférence NRRL 18349, NRRL 18350 ou NRRL 18364.

**3.** Préparation enzymatique selon la revendication 1 ou 2, ayant un optimum de pH dans l'intervalle 7 - 8, et ayant une activité à pH 9 qui est au moins égale à 80 % de l'activité à pH 7,5.

**4.** Préparation enzymatique selon l'une quelconque des revendications précédentes, caractérisée en outre par sa capacité à hydrolyser les parois cellulaires bactériennes de *Micrococcus kristinae*.

**5.** Préparation enzymatique selon la revendication 4, dans laquelle la souche de *Nocardiopsis* est *N. dassonvillei* NRRL 18349.

**6.** Préparation enzymatique selon la revendication 5, comprenant une enzyme lytique ayant une masse moléculaire de 24 000 ou 26 000 et respectivement un point isoélectrique de 8,3 ou d'au moins 9,5.

**7.** Préparation enzymatique selon l'une quelconque des revendications précédentes, qui comprend en outre une protéase alcaline de *Bacillus*.

**8.** Préparation enzymatique mixte selon la revendication 7, comprenant de 50 000 à 1 million d'unités d'enzyme bactériolytique par gramme d'additif et de 0,5 à environ 3,0 unités Anson de protéase par gramme d'additif.

**9.** Préparation enzymatique selon l'une quelconque des revendications précédentes, sous forme d'un additif de détergent, de préférence sous forme d'un granulé ne formant pas de poussière ou d'un liquide stabilisé.

17

**10.** Procédé de production de l'enzyme bactériolytique selon l'une quelconque des revendications précédentes, qui comprend la culture d'une souche de *Nocardiopsis* productrice d'une enzyme bactériolytique, dans des conditions aérobies, dans un milieu nutritif contenant des sources assimilables de carbone, d'azote et de phosphore, puis la récupération de l'enzyme à partir du bouillon de culture.

**11.** Procédé selon la revendication 10, dans lequel la souche appartient à *N. dassonvillei.*

**12.** Procédé selon la revendication 11, dans lequel la souche est NRRL 18349, NRRL 18350, NRRL 18364 ou un mutant ou une variante de ces souches.

**13.** Culture biologiquement pure d'une souche de *Nocardiopsis* capable de produire l'enzyme bactériolytique de la revendication 1, 3, 4 ou 6.

**14.** Culture selon la revendication 13, dans laquelle la souche appartient à *N. dassonvillei.*

**15.** Culture selon la revendication 14 de la souche NRRL 18349, NRRL 18350, NRRL 18364 ou d'un mutant ou d'une variante de ces souches.

**16.** Composition détergente comprenant une préparation d'enzyme bactériolytique selon l'une quelconque des revendications 1 à 9.

**17.** Composition détergente selon la revendication 16, comprenant 1000 à 20 000 unités d'enzyme bactériolytique par gramme de détergent.

**18.** Composition détergente selon la revendication 16 ou 17, comprenant en outre 0,001 à 0,5 unité Anson de protéase alcaline de *Bacillus* par gramme de détergent.

**19.** Utilisation de la préparation enzymatique selon l'une quelconque des revendications 1 à 9 pour l'hydrolyse de parois cellulaires bactériennes.

**20.** Utilisation selon la revendication 19, pour la réduction du nombre de bactéries nuisibles.

**21.** Utilisation selon la revendication 20 comme conservateur alimentaire, pour la désinfection dans le traitement des aliments, pour le traitement de l'eau, pour la désinfection d'instruments hospitaliers ou pour la réduction de l'odeur corporelle dans les vêtements.

**22.** Utilisation selon la revendication 20 pour la réduction de l'odeur corporelle dans les vêtements par lavage ou rinçage des vêtements dans une eau de lavage contenant un détergent ou dans une eau de rinçage sans détergent contenant la préparation enzymatique.

**23.** Utilisation selon l'une quelconque des revendications 19 à 22, dans laquelle l'eau de lavage ou de rinçage contient 1000 - 20 000 unités d'enzyme bactériolytique par litre.

**24.** Utilisation selon l'une quelconque des revendications 19 à 23, dans laquelle l'eau de lavage ou de rinçage contient en outre une protéase alcaline de *Bacillus*, de préférence à un niveau d'activité de 0,02 à 0,15 unités Anson par litre.

**25.** Utilisation selon la revendication 19, pour le traitement de masses cellulaires bactériennes.

**26.** Utilisation selon la revendication 25, pour le traitement de boues activées, pour la formation de protoplastes ou pour la récupération de composés sécrétés par voie intracellulaire.

**27.** Déodorant corporel comprenant une préparation enzymatique bactériolytique selon l'une quelconque des revendications 1 à 9.

# FIG. 1.

THE pH-ACTIVITY PROFILE OF CRUDE LYTIC ENZYME FROM N. DASSONVILLEI STRAIN G102-3